(19) Europäisches Patentamt
European Patent Office
Office européen des brevets

(11) EP 1 862 109 A2

(12) **EUROPEAN PATENT APPLICATION**

(43) Date of publication:
**05.12.2007 Bulletin 2007/49**

(51) Int Cl.:
***A61B 1/05*** *(2006.01)* ***A61B 5/07*** *(2006.01)*
*A61B 5/00* *(2006.01)*

(21) Application number: **07010819.6**

(22) Date of filing: **31.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **01.06.2006 JP 2006153277**

(71) Applicant: **FUJIFILM Corporation**
**Minato-ku**
**Tokyo (JP)**

(72) Inventor: **Tsujita, Kazuhiro**
**Minato-ku**
**Tokyo (JP)**

(74) Representative: **Klunker . Schmitt-Nilson . Hirsch**
**Winzererstrasse 106**
**80797 München (DE)**

(54) **Capsule endoscopic system and image processing apparatus**

(57) A capsule endoscope (11, 91) for endoscopic imaging is swallowable in a body (10), for image pickup of an object. In a capsule endoscopic system, a receiver (12) receives image data in a form of a radio signal from the capsule endoscope, and for storing the image data. A computer as image processor produces an object image (81) according to the image data from the receiver. A spectral image generator (69) produces data of a spectral image (82) having a wavelength band by arithmetic operation according to the image data. To this end, the spectral image generator arithmetically operates with a coefficient matrix (67) to form the spectral image. Furthermore, capsule type information (72), assigned to the capsule endoscope discretely, is retrieved. The capsule type information is stored in a data storage medium (64), and read by the spectral image generator to determine the coefficient matrix.



Printed by Jouve, 75001 PARIS (FR)

## Description

### BACKGROUND OF THE INVENTION

#### 1. Field of the Invention

The present invention relates to a capsule endoscopic system and image processing apparatus. More particularly, the present invention relates to a capsule endoscopic system for endoscopic imaging of a digestive system by use of radio transmission of image data of a capsule endoscope, and image processing apparatus for use therein.

#### 2. Description Related to the Prior Art

In the field of medical imaging, a capsule endoscope has been developed. The capsule endoscope is a very small unit, swallowable, and includes an image pickup device and a light source. For diagnosis, at first a patient swallows the capsule endoscope. The light source of the capsule endoscope in the human body of the patient applies light to an object in a tract of the human body, for example gastrointestinal tract. The image pickup device photographs the object with the light, to obtain image data. There is a receiver as communication interface, disposed extracorporeally, and receives the radio transmission of the image data. The image data transmitted from the capsule endoscope is stored in a flash memory or other data storage medium loaded in the receiver. After the diagnosis, the image data from the receiver is retrieved in a computer or an image processor, which causes a display panel to display an object image.

One technique referred to as Narrow Band Imaging (NBI) has been known recently in the field of endoscopic imaging. Light of a narrow band is applied to the object, so that reflected light is imaged as a spectral image, and observed for the purpose of easy discovery of a lesion in a tissue. The spectral image is a newer form distinct from the object image. It is unnecessary in the NBI to disperse dye on to the object, or injecting indocyanine green (ICG) as contrast agent. It is possible easily to retrieve an image in which blood vessel of a submucosa is emphasized, or an image of a wall of a gastrointestinal tract of which a structure is emphasized.

U.S.P. 7,153,259 (corresponding to JP-A 2005-074034) discloses application of the NBI in the capsule endoscope. The capsule endoscope has a white light source for emitting white light and a narrow band light source for emitting narrow band light. The white light source and the narrow band light source are driven successively to emit light. The object being illuminated is photographed by the image pickup device one frame after another with the reflected light. The capsule endoscope has a filter, disposed in a light path at one of the white light source and the image pickup device, for optically transmitting the narrow band light.

However, the structure of the above document has a shortcoming in the complicated sequence of driving due to the plurality of the light source. The size and the cost of the capsule endoscope may be larger. The power required for operation may be too high.

The spectral image has a quality defined uniquely by the characteristics of the filter. There is no technique of forming the spectral image by considering specific differences between products of the capsule endoscope, or considering differences between various uses for object types, for example esophagus, stomach, and small and large intestines. Even in one imaging system, the number of product types of the capsule endoscope is high as well as the number of products of each type of the capsule endoscope in use is high. However, due to the lack of consideration of specific differences, it is likely in the diagnosis that reliable results are unavailable.

### SUMMARY OF THE INVENTION

In view of the foregoing problems, an object of the present invention is to provide a capsule endoscopic system for endoscopic imaging of a digestive system by use of radio transmission of image data of a capsule endoscope, and in which a spectral image is available easily, and image processing apparatus for use therein.

In order to achieve the above and other objects and advantages of this invention, a capsule endoscopic system for endoscopic imaging includes a capsule endoscope, swallowable in a body, for image pickup of an object. A receiver receives image data in a form of a radio signal from the capsule endoscope, and for storing the image data. An image processor produces an object image according to the image data from the receiver. A spectral image generator produces data of a spectral image having a wavelength band by arithmetic operation according to the image data.

The spectral image generator arithmetically operates with a coefficient matrix to obtain the data of the spectral image according to the image data.

Furthermore, a specific information retriever retrieves specific information assigned to the capsule endoscope discretely. A data storage medium stores the coefficient matrix in association with the specific information, and operates for access by the spectral image generator according to the specific information from the specific information retriever.

The specific information includes a capsule type number and production lot number of the capsule endoscope.

The capsule endoscope includes a memory for storing the specific information. A radio transmission unit transmits the specific information to the receiver in a form of a radio signal.

The radio transmission unit transmits the specific information upon turning on of a power source thereof.

Furthermore, a position detector detects a position of the capsule endoscope in the body. A data storage medium stores the coefficient matrix in association with the object, and operates for access by the spectral image generator according to a detection result of the position detector.

The position detector includes an electric field strength detector for measuring an electric field strength of an electric field of the radio signal.

The receiver includes plural antennas, positioned on the body, connected with the electric field strength detector, for receiving the radio signal.

In a preferred embodiment, the position detector includes a magnet associated with the capsule endoscope. A magnetic field sensor measures a magnetic field strength of a magnetic field created by the magnet.

The magnetic field sensor is associated with the receiver, and positioned on the body.

Furthermore, a specific information retriever retrieves specific information assigned to the capsule endoscope discretely. The data storage medium stores the coefficient matrix in association with the specific information and the object, and is accessed by the spectral image generator according to a combination of the specific information from the specific information retriever and a detection result of the position detector.

Furthermore, a spectrum measuring device measures a spectrum of the spectral image. A display control unit operates for display of information of a measuring result of the spectrum measuring device in addition to the spectral image.

An attention indicia is caused by the display control unit to appear with the spectral image, and indicates a feature region derived from the measuring result of the spectrum measuring device.

Furthermore, a capsule holder supports the capsule endoscope before ingestion in the body. A test image is positioned on a holder surface of the capsule holder opposed to the capsule endoscope. A matrix determiner determines the coefficient matrix according to test image data obtained by photographing the test image by the capsule endoscope.

The test image is a test color chart including plural regions with colors different from one another.

The capsule endoscope includes a light source for applying illuminating light to the object. An image pickup device detects object light of the object. An optical system focuses the object light on the image pickup device in application of the illuminating light to the object. A radio transmission unit transmits the image data of the object light to the receiver in a form of the radio signal according to an output of the image pickup device.

In another preferred embodiment, an image processor for outputting an object image, by image processing according to image data of endoscopic imaging obtained from a capsule endoscope, is provided. The image processor includes a spectral image generator for producing data of a spectral image having a wavelength band by arithmetic operation according to the image data.

The spectral image generator arithmetically operates with a coefficient matrix to obtain the data of the spectral image according to the image data.

The capsule endoscope includes a memory for storing the specific information. A radio transmission unit transmits the specific information in a form of a radio signal. Furthermore, an object image generator produces the object image according to the image data received by a communication interface from the capsule endoscope with the radio signal.

Also, a computer executable program for endoscopic imaging is provided, in which a capsule endoscope is used and swallowable in a body, for image pickup of an object. The computer executable program includes an image generating program code for producing an object image according to image data received in a form of a radio signal from the capsule endoscope with a communication interface. A spectral image generating program code is for producing a spectral image having a wavelength band by arithmetic operation according to the image data.

Also, a user interface for endoscopic imaging is provided, in which a capsule endoscope is used and swallowable in a body, for image pickup of an object. The user interface includes an image display region for displaying an object image according to image data received in a form of a radio signal from the capsule endoscope with a communication interface. A spectral image display region is for displaying a spectral image having a wavelength band by arithmetic operation according to the image data.

Consequently, a spectral image can be formed easily, because wirelessly transmitted image data of a capsule endoscope is subjected to arithmetic operation to obtain spectral image data for endoscopic imaging.

## BRIEF DESCRIPTION OF THE DRAWINGS

The above objects and advantages of the present invention will become more apparent from the following detailed description when read in connection with the accompanying drawings, in which:

Fig. 1A is an explanatory view in elevation illustrating a body of a patient and a capsule endoscope during ingestion into a digestive tract;

Fig. 1B is a perspective view illustrating a capsule endoscope system in which a receiver is connected to a computer;

Fig. 2 is a cross section illustrating the capsule endoscope;

Fig. 3 is a block diagram schematically illustrating circuits in the capsule endoscope;

Fig. 4 is a block diagram schematically illustrating the receiver;

Fig. 5 is a block diagram schematically illustrating the computer as image processor;

Fig. 6 is a schematic view illustrating a display screen of the monitor display panel;

Fig. 7 is an explanatory view in elevation illustrating another preferred capsule endoscope having a magnet, in combination with Hall elements at antennas;

Fig. 8 is a block diagram schematically illustrating one preferred embodiment of computer as image processor;

Fig. 9 is a schematic view illustrating a display screen with the spectral image, measured information and attention indicia;

Fig. 10 is a front elevation illustrating a capsule holder for the capsule endoscope;

Fig. 11 is a plan illustrating a test color chart for determining a coefficient matrix; and

Fig. 12 is a block diagram schematically illustrating a computer for use in operation with the test color chart.

## DETAILED DESCRIPTION OF THE PREFERRED EMBODIMENT(S) OF THE PRESENT INVENTION

In Figs. 1A and 1B, a capsule endoscope system 2 for endoscopic imaging includes a capsule endoscope 11 or endoscopic imaging capsule, a receiver 12 or communication interface, and a computer 13 as image processor. The capsule endoscope 11 is swallowed orally by a patient, and passes through a gastrointestinal lumen in a digestive tract of a human body 10 of the patient. The capsule endoscope 11 picks up a wall of a lumen, obtains image data, and wirelessly transmits the image data.

For the diagnosis, the human body 10 wears shielding clothes 8. A plurality of antennas 14 of the receiver 12 or communication interface are disposed on an inner surface of the shielding clothes 8. The receiver 12 wirelessly receives image data from the capsule endoscope 11 by use of the antennas 14. A data storage medium 48 of the receiver 12 in Fig. 4 is caused to store the image data. A front of the receiver 12 is provided with an LCD display panel 15 and a button panel 16. The display panel 15 displays any of various menu regions for settings. The button panel 16 is operable for inputs. The receiver 12 is connected with the computer 13 by a communication cable such as a USB cable for transmitting and receiving data and signals.

The computer 13 as image processor has an input interface 17 such as a keyboard and mouse and a monitor display panel 18. When the diagnosis with the capsule endoscope 11 is completed, the computer 13 retrieves image data from the data storage medium 48 in the receiver 12 or communication interface, produces an object image, and causes the display panel 18 to display the object image.

An electric field strength detector 19 or electric field sensor is incorporated in the antennas 14. When radio wave 36 of Fig. 3 is emitted by the capsule endoscope 11, the electric field strength detector 19 measures the electric field of the radio wave 36. A position detector 43 of Fig. 4 is supplied with an output of the electric field strength detector 19 as a result of the measurement.

In Fig. 2, the capsule endoscope 11 includes a transparent front casing 20 and a rear casing 21 fitted on the transparent front casing 20 in a watertight manner to enclose an inner space. Each of the front and rear casings 20 and 21 is in a combined shape including a tubular portion of a cylindrical form and an end portion of a semispherical form.

Various elements are contained in a space inside the front and rear casings 20 and 21, including a camera head or optical assembly 24 for imaging, a light source 25, a circuit board 26 and a button cell battery 27. The camera head 24 includes an objective optical system 22 for passing of object light from an object of the endoscopic imaging, and an image pickup device 23 such as a CCD, CMOS or other imaging element. The light source 25 is a white LED (light emitting diode) for illuminating the object. The circuit board 26 has a radio transmission unit 35 and a power source circuit 37 mounted thereon in Fig. 3. An antenna 28 is disposed on the rear of the circuit board 26, and connected with the radio transmission unit 35 of Fig. 3.

The objective optical system 22 includes an optical dome or transparent cover 22a, a lens holder 22b, and a lens 22c. The transparent cover 22a is disposed at a cover end of the transparent front casing 20 in a semi spherical shape. The lens holder 22b is secured to a rear end of the transparent cover 22a, and has a width decreasing in the backward direction. The lens 22c is supported in the lens holder 22b. There is an optical axis 29 of the objective optical system 22, which has a photographic field region of a view angle of 140-180 degrees. Image light of an image of an intraluminal object is retrieved in all directions in the field region.

In Fig. 3, a CPU 30 controls the entirety of the capsule endoscope 11. A ROM 31 is connected with the CPU 30, and stores various programs and data for control of the capsule endoscope 11. The CPU 30 reads a program or data from the ROM 31 when required, for control of the capsule endoscope 11.

A driver 32 is connected with the CPU 30. As image light is focused on a pickup surface of the image pickup device 23, the CPU 30 causes the driver 32 to output a pickup signal of pixels at a frame rate of 2 frames per second or the like.

An AFE (analog front end) 33 is controlled by use of the driver 32, and receives an image signal from the image pickup device 23. The AFE 33 processes the image signal according the correlated double sampling, amplification, and A/D conversion, and outputs a digital image data of 10 bits by conversion of the image signal.

A modulator 34 modulates the image data from the AFE 33 according to the digital quadrature modulation, and produces the RF signal. The radio transmission unit 35 transmits the radio wave 36 of the RF signal from the modulator 34 by use of the antenna 28 to the receiver 12 or communication interface.

The power source circuit 37 is caused by the CPU 30 to supply various elements of the capsule endoscope 11 with power from the button cell battery 27. An illumination driver 38 is controlled by the CPU 30, and turns on and off the light source 25.

The ROM 31 stores specific information of the capsule endoscope 11, such as the capsule type number of the capsule endoscope 11 for the large intestinal use, small intestinal use and other uses, and a production lot number of the capsule endoscope 11. When the power source of the capsule endoscope 11 is turned on, the CPU 30 reads the specific information from the ROM 31, and outputs the same to the radio transmission unit 35. The radio transmission unit 35 transmits the radio wave 36 of the specific information from the CPU 30 by use of the antenna 28 to the receiver 12.

In Fig. 4, a CPU 40 controls the entirety of the receiver 12 or communication interface. A ROM 41 is connected with the CPU 40, and stores various programs and data for control of the receiver 12. The CPU 40 reads a program or data from the ROM 41 when required, for control of the receiver 12. Also, the CPU 40 responds to input signals generated by depressing the button panel 16, and causes the receiver 12 to operate.

A reception unit 42 amplifies an RF signal or a signal of the radio wave 36 received by the antennas 14. Also, the reception unit 42 receives the radio wave 36 of specific information upon turning on of the power of the capsule endoscope 11, and sends the specific information to the CPU 40.

The position detector 43 detects a position of the capsule endoscope 11 in the body 10 according to the measured electric field of the radio wave 36 detected by the electric field strength detector 19, and transmits the position information to the CPU 40. Specifically for the position detection of the capsule endoscope 11, for example, data of a data table is experimentally obtained and written in the ROM 41, the data table expressing a relationship between the electric field of the radio wave 36 at the antennas 14 and a position of the capsule endoscope 11 in the body 10. The ROM 41 is accessed to refer to the data table, for determining the position.

A demodulator 44 receives the RF signal in the form of the radio wave 36, and demodulates the RF signal according to the digital quadrature detection to obtain image data before demodulation in the capsule endoscope 11. A sync separator 45 is controlled by the CPU 40, separates the sync signal from the demodulated image data from the demodulator 44 according to the amplitude separation. Then the sync separator 45 separates horizontal sync signal and vertical sync signal of the image data according to the frequency separation.

A digital signal processor (DSP) 46 processes the demodulated image data from the demodulator 44 according to signal processing of gamma processing, Y/C processing and the like. A luminance signal and a chrominance signal in combination are transmitted to a memory control unit 47 as image data. The memory control unit 47 combines the specific information and position information with the image data output by the digital signal processor 46, and writes the combination of the data to the data storage medium 48. An example of the data storage medium 48 is a flash memory having a storage size as much as 1 Gb, and stores numerous image data output by the memory control unit 47.

A display driver 49 drives the LCD display panel 15 in a controlled manner. A connector 51 is connectable with a USB cable or the like. A communication interface port 50 sends data to or receives data from the computer 13 as image processor in connection with the connector 51. Image data read from the data storage medium 48 is transmitted by the interface port 50 to the computer 13.

In Fig. 5, a CPU 60 controls the entirety of the computer 13 as image processor. Circuit elements are connected with the CPU 60, including a display control unit 61, a communication interface 63, a hard disk drive or HDD 64 as data storage medium, and a memory 65. The display control unit 61 controls the monitor display panel 18. The communication interface 63 is connectable by use of a connector 62, and receives image data from the receiver 12 or communication interface.

An image processing program 66 and data of coefficient matrices 67 are stored in the HDD 64 together with programs and data required for operation of the computer 13 as image processor. Also, image data received through the communication interface 63 is stored in the HDD 64. The memory 65 for a temporary use stores data read from the HDD 64, intermediate data produced during the arithmetic operation, and the like.

When the image processing program 66 is run by operating the input interface 17, a working window of the image processing program 66 appears on the monitor display panel 18. The input interface 17 is operated by a user, to display or edit an object image. Also, an object image generator 68 for an object image and a spectral image generator 69 become ready for operation in the CPU 60 upon startup of the image processing program 66.

The object image generator 68 reads image data from the HDD 64, and generates normal image data or object image data according to the image data. Also, the spectral image generator 69 receives the image data, and produces spectral image data of a spectral image having a wavelength band. The wavelength band is changeable by operating the input interface 17.

A matrix operating unit 70 is incorporated in the spectral image generator 69. The matrix operating unit 70 reads the coefficient matrix 67 from the HDD 64, and carries out the matrix arithmetic operation to multiply the image data by the coefficient matrix 67. The arithmetic operation is expressed in the following equation

$$C A = C'$$

where C is a matrix of the image data, C' is a matrix of the spectral image data, and A is a coefficient matrix.

Note that the coefficient matrix 67 is a group of predetermined coefficients for the purpose of converting image data into spectral image data of a predetermined wavelength band in accordance with the equation.

$$A = (tC\ C)^{-1}\ tC\ C'$$

where tC is a transposed matrix of the matrix C. As the coefficient matrix 67 depends upon characteristics of color filters of the image pickup device 23 or spectral characteristics of light from the light source 25, spectral reflection characteristics of objects to be imaged. The coefficient matrix 67 requires correcting for the purpose of optimizing a spectral image. Thus, the HDD 64 stores data table of various data including the capsule type number of the capsule endoscope 11 to express intracorporeal regions in the endoscopic use, the production lot number of the image pickup device 23 and the light source 25, and a plurality of the coefficient matrices 67 according to the intracorporeal regions. The matrix operating unit 70 reads the coefficient matrix 67 from the HDD 64 in association with specific information 72 such as a capsule type number and production lot number of the capsule endoscope 11 in association with the image data, and position information. The matrix operating unit 70 performs calculation for matrix operation by use of the coefficient matrix 67.

In Fig. 6, the display control unit 61 outputs information to cause the monitor display panel 18 to display an object image 81 according to the object image data, and a spectral image 82 according to the spectral image data in combination with personal information 80 of a patient or diagnosis date. Note that it is possible to display any one of the object image 81 and the spectral image 82 at one time on the display panel 18.

Operation of diagnosis by use of the capsule endoscope system 2 for endoscopic imaging is described now. At first, power starts being supplied to the capsule endoscope 11. The specific information 72 is read from the ROM 31 by the CPU 30, which sends the specific information 72 to the radio transmission unit 35. The radio transmission unit 35 transmits the radio wave 36 of the specific information 72 by use of the antenna 28 to the receiver 12 or communication interface.

The patient swallows the capsule endoscope 11 orally. An object in the body 10 is illuminated by the light source 25 in the digestive system, while the image pickup device 23 photographs the inner wall of the tract. Object light of the object becomes incident on the objective optical system 22, and is focused on a pickup surface of the image pickup device 23, to output an image signal from the image pickup device 23. The AFE 33 processes the image signal from the image pickup device 23 according the correlated double sampling, amplification, and A/D conversion, and outputs digital image data of 10 bits by conversion.

The digital image data from the AFE 33 is modulated by the digital quadrature modulation in the modulator 34, to generate an RF signal. The RF signal is amplified by the radio transmission unit 35 to transmit the radio wave 36 by use of the antenna 28.

When the antennas 14 of the receiver 12 or communication interface receive the radio wave 36 output by the antenna 28 of the capsule endoscope 11, the RF signal or the received radio wave is amplified by the reception unit 42. Also, the electric field of the radio wave 36 is measured by the electric field strength detector 19. An intracorporeal position of the capsule endoscope 11 is detected by the position detector 43 according to the output of the electric field strength detector 19, so as to send the position information to the CPU 40.

The RF signal amplified by the reception unit 42 is subjected by the demodulator 44 to digital quadrature detection, and is demodulated to a form of the image data before modulation in the capsule endoscope 11. The demodulated image data is subjected to sync separation by the sync separator 45 conditioned by the CPU 40. The signal is processed in the digital signal processor 46 in various steps of signal processing. Then the specific information 72 from the memory control unit 47 and the position information are assigned to the image data, and stored in the data storage medium 48.

After the diagnosis with the capsule endoscope 11, the receiver 12 or communication interface is connected to the computer 13 as image processor. Image data in the data storage medium 48 is transmitted by the interface port 50, the connector 51, the USB cable and the connector 62, and received by the communication interface 63 of the computer 13. The image data is written to the HDD 64.

When the image processing program 66 is run by operating the input interface 17, the monitor display panel 18 at the computer 13 as image processor displays a working window region for the image processing program 66. The object image generator 68 and the spectral image generator 69 are started up in the CPU 60. The object image generator 68 reads image data from the HDD 64, and produces object image data.

The spectral image generator 69 as specific information retriever reads the specific information 72 and the coefficient matrix 67 from the HDD 64, the specific information 72 being associated with the image data, the coefficient matrix 67 being associated with the position information. The specific information 72 and the coefficient matrix 67 are retrieved in the matrix operating unit 70. The matrix operating unit 70 processes the image data for arithmetic operation of the coefficient matrix 67 read from the HDD 64. The matrix operating unit 70 produces spectral image data. The object image data and the spectral image data are received by the display control unit 61 which causes the monitor display panel 18 to display the object image 81 and the spectral image 82.

Accordingly, a spectral image can be formed in the capsule endoscope system 2 for endoscopic imaging by the simplified construction without using plural light sources or plural filters of narrow bands, because the spectral image data is produced from image data by the spectral image generator 69.

To produce the spectral image, the coefficient matrix 67 of an optimized condition for the specific information 72 and the position information is used. Thus, a good spectral image can appear without differences of the capsule endoscope 11 between plural products, and without differences between objects of the diagnosis.

Also, the specific information 72 is automatically transmitted from the capsule endoscope 11 to the receiver 12 or communication interface wirelessly as soon as the power for the capsule endoscope 11 is turned on. This is effective in reducing the manufacturing cost because there is no keypad for an operator to operate to obtain the specific information 72.

In the above embodiment, the electric field strength detector 19 is used for position detection. In Fig. 7, another preferred embodiment is illustrated, in which magnetic field measuring Hall elements 92 are used. A capsule endoscope 91 includes a magnet 90 to detect a position, and antennas 93. The Hall elements 92 are associated with the antennas 93, and measure strength of a magnetic field of the magnet 90. The result of the measurement is evaluated in the position detector 43 to detect the position of the capsule endoscope 11 in the body 10. Alternatively, a technique of image recognition can be utilized in place of the electric field strength detector 19 or the Hall elements 92. An image recognition unit is provided in the computer 13 as image processor, to detect a position of the object by analysis of the image data from the capsule endoscope 11.

In Fig. 8, another preferred embodiment is illustrated in which a spectrum measuring device 100 is included in the CPU 60. The spectrum measuring device 100 measures a spectrum for each of plural regions defined by regularly splitting the image frame in an expressed form of spectral image data of the regions.

In Fig. 9, the display control unit 61 causes the monitor display panel 18 to display result information 101 of the measurement of the spectrum measuring device 100 in combination with the object image 81 and the spectral image 82. The result information 101 includes a spectral graph 101a and numerical data 101b. Also, a frame pattern 102 as attention indicia is indicated with the spectral image 82. A position of the frame pattern 102 is extracted from the measurement of the spectrum measuring device 100. Examples of the frame pattern 102 are a region with a particularly different spectrum from remaining regions, an area with a spectrum with an estimated lesion according to past data of the disease and the like. It is possible to diagnose the patient rapidly and reliably by indication of the result information 101 and the frame pattern 102.

In the above embodiment, various values of the coefficient matrices 67 are predetermined. However, the coefficient matrix 67 with a change may be corrected for compensation in view of a characteristic change with time after the shipment. Also, a new value of the coefficient matrix 67 may be determined according to such a characteristic change with time. In Fig. 10, a capsule holder 110 or stand is prepared for an unused body of the capsule endoscope 11 before diagnosis. A holder recess 111 is formed in the capsule holder 110 for supporting the capsule endoscope 11 with the camera head or optical assembly 24 directed downwards. In Fig. 11, an inner holder surface 112 of the holder recess 111 has a test color chart 113 as test image for determining a matrix. Eight color regions 113a of an equal area are defined by splitting a circular region, and have colors of red, green, blue, cyan, magenta, yellow, black and white. In Fig. 12, a matrix determiner 114 is included in the CPU 60.

In Figs. 10-12 , the correction or renewal of the coefficient matrix 67 is depicted. At first, the capsule endoscope 11 before the diagnosis is fitted in the holder recess 111 in the capsule holder 110 by directing down the camera head or optical assembly 24. The power source in the capsule endoscope 11 is turned on to photograph the test color chart 113. Test image data of the test color chart 113 are obtained by the capsule endoscope 11, which transmits the radio wave 36 of the test image data to the receiver 12.

Then the receiver 12 or communication interface is connected to the computer 13 as image processor, and is caused to send the test image data to the computer 13. The matrix determiner 114 receives the test image data, and corrects the coefficient matrix 67 by considering a change in the coefficient matrix 67 according to characteristic changes in the capsule endoscope 11 with time. It is also possible in the matrix determiner 114 to generate a new coefficient matrix according to changes with time. Various methods can be used for correction or renewal of a coefficient matrix. For example, reference test image data of the test image data is obtained experimentally with the test color chart 113 as test image before shipment of the product, and stored in the HDD 64. The test image data from the receiver 12 is compared with the reference test image data, to obtain difference data. The coefficient matrix 67 is corrected according to the difference data. Also, a new coefficient matrix may be obtained according to the difference data. Therefore, optimized spectral image data can be obtained even when a change occurs in the characteristic of the capsule endoscope 11 after the shipment.

In the above embodiment, both the specific information 72 and the position information are referred to for the purpose of producing a spectral image. However, only a selected one of the specific information 72 and the position information can be used for producing a spectral image.

In the above embodiment, the image processing program 66 is installed in the HDD 64 in the computer 13 as image processor, and is run to function the object image generator 68 and the spectral image generator 69 in the CPU 60. However, elements of the object image generator 68, the spectral image generator 69 and the like may be discrete circuits, FPGA (Field Programmable Gate Array), and other circuits of hardware.

In the above embodiment, the capsule endoscope 11 is a terminal device for use of only transmission without reception. However, the capsule endoscope 11 can be constructed for both transmission and reception of a signal or data. A power turning-on signal may be wirelessly sent to the capsule endoscope 11, so as to turn on the power source of the capsule endoscope 11.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

**1.** A capsule endoscopic system for endoscopic imaging, comprising:

a capsule endoscope (11, 91), swallowable in a body (10), for image pickup of an object;
a receiver (12) for receiving image data in a form of a radio signal from said capsule endoscope, and for storing said image data;
an image processor (13, 60, 61) for producing an object image (81) according to said image data from said receiver; and
a spectral image generator (69) for producing data of a spectral image (82) having a wavelength band by arithmetic operation according to said image data.

**2.** A capsule endoscopic system as defined in claim 1, wherein said spectral image generator arithmetically operates with a coefficient matrix to obtain said data of said spectral image according to said image data.

**3.** A capsule endoscopic system as defined in claim 2, further comprising:

a specific information retriever for retrieving specific information assigned to said capsule endoscope discretely;
a data storage medium for storing said coefficient matrix in association with said specific information, and for access by said spectral image generator according to said specific information from said specific information retriever.

**4.** A capsule endoscopic system as defined in claim 3, wherein said specific information includes a capsule type number and production lot number of said capsule endoscope.

**5.** A capsule endoscopic system as defined in claim 3, wherein said capsule endoscope includes:

a memory for storing said specific information; and
a radio transmission unit for transmitting said specific information to said receiver in a form of a radio signal.

**6.** A capsule endoscopic system as defined in claim 5, wherein said radio transmission unit transmits said specific information upon turning on of a power source thereof.

**7.** A capsule endoscopic system as defined in claim 2, further comprising:

a position detector for detecting a position of said capsule endoscope in said body;
a data storage medium for storing said coefficient matrix in association with said object, and for access by said spectral image generator according to a detection result of said position detector.

**8.** A capsule endoscopic system as defined in claim 7, wherein said position detector includes an electric field strength detector for measuring an electric field strength of an electric field of said radio signal.

**9.** A capsule endoscopic system as defined in claim 8, further comprising plural antennas, positioned on said body, connected with said electric field strength detector, for receiving said radio signal.

**10.** A capsule endoscopic system as defined in claim 7, wherein said position detector includes:

a magnet associated with said capsule endoscope;
a magnetic field sensor for measuring a magnetic field strength of a magnetic field created by said magnet.

**11.** A capsule endoscopic system as defined in claim 10, further comprising plural antennas, positioned on said body, connected with said magnetic field sensor, for receiving said radio signal.

**12.** A capsule endoscopic system as defined in claim 7, further comprising a specific information retriever for retrieving specific information assigned to said capsule endoscope discretely;
wherein said data storage medium stores said coefficient matrix in association with said specific information and said object, and is accessed by said spectral image generator according to a combination of said specific information from said specific information retriever and a detection result of said position detector.

**13.** A capsule endoscopic system as defined in claim 12, wherein said position detector includes an electric field strength detector for measuring an electric field strength of an electric field of said radio signal.

**14.** A capsule endoscopic system as defined in claim 13, further comprising plural antennas, positioned on said body, connected with said electric field strength detector, for receiving said radio signal.

**15.** A capsule endoscopic system as defined in claim 12, wherein said position detector includes:

a magnet associated with said capsule endoscope;
a magnetic field sensor for measuring a magnetic field strength of a magnetic field created by said magnet.

**16.** A capsule endoscopic system as defined in claim 15, further comprising plural antennas, positioned on said body, connected with said magnetic field sensor, for receiving said radio signal.

**17.** A capsule endoscopic system as defined in claim 1, further comprising:

a spectrum measuring device for measuring a spectrum of said spectral image; and
a display control unit for display of information of a measuring result of said spectrum measuring device with said spectral image.

**18.** A capsule endoscopic system as defined in claim 17, wherein an attention indicia is caused by said display control unit to appear with said spectral image, and indicates a feature region derived from said measuring result of said spectrum measuring device.

**19.** A capsule endoscopic system as defined in claim 1, further comprising:

a capsule holder for supporting said capsule endoscope before ingestion in said body, said capsule holder having a test image on a surface thereof to acquire said coefficient matrix;
a matrix determiner for determining said coefficient matrix according to test image data obtained by photographing said test imaged by said capsule endoscope.

**20.** A capsule endoscopic system as defined in claim 19, wherein said test image is constituted by a test color chart including plural regions disposed at a regular pitch with colors different from one another.

**21.** An image processor for outputting an object image (81) by image processing according to image data of endoscopic imaging obtained from a capsule endoscope (11, 91), comprising:

a spectral image generator (69) for producing data of a spectral image (82) having a wavelength band by arithmetic operation according to said image data.

**22.** An image processor as defined in claim 21, wherein said spectral image generator arithmetically operates with a coefficient matrix to obtain said data of said spectral image according to said image data.

**23.** An image processor as defined in claim 22, further comprising:

a specific image retriever for retrieving specific information assigned to said capsule endoscope discretely; and
a data storage medium for storing said coefficient matrix in association with said specific information, and for access by said spectral image generator according to said specific information from said specific image retriever.

**24.** An image processor as defined in claim 23, wherein said specific information includes a capsule type number and production lot number of said capsule endoscope.

**25.** An image processor as defined in claim 22, further comprising:

a position detector for detecting a position of said capsule endoscope in said body;
a data storage medium for storing said coefficient matrix in association with said object, and for access by said spectral image generator according to a detection result of said position detector.

**26.** An image processor as defined in claim 25, further comprising a specific information retriever for retrieving specific information assigned to said capsule endoscope discretely;
wherein said data storage medium stores said coefficient matrix in association with said specific information and said object, and is accessed by said spectral image generator according to a combination of said specific information from said specific information retriever and a detection result of said position detector.

**27.** An image processor as defined in claim 22, further comprising:

a spectrum measuring device for measuring a spectrum of said spectral image; and
a display control unit for display of information of a measuring result of said spectrum measuring device with said spectral image.

**28.** An image processor as defined in claim 27, wherein an attention indicia is caused by said display control unit to appear with said spectral image, and indicates a feature region derived from said measuring result of said spectrum measuring device.

**29.** An image processor as defined in claim 22, further comprising:

a capsule holder for supporting said capsule endoscope before ingestion in said body, said capsule holder having a test image on a surface thereof to acquire said coefficient matrix;
a matrix determiner for determining said coefficient matrix according to test image data obtained by photographing said test image by said capsule endoscope.

**30.** An image processor as defined in claim 29, wherein said test image is constituted by a test color chart including-plural regions disposed at a regular pitch with colors different from one another.

FIG. 1A

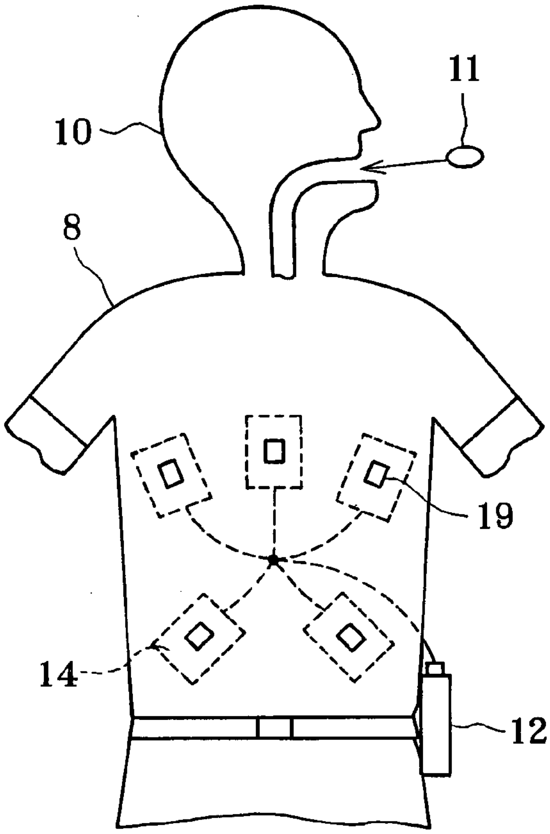


FIG. 1B

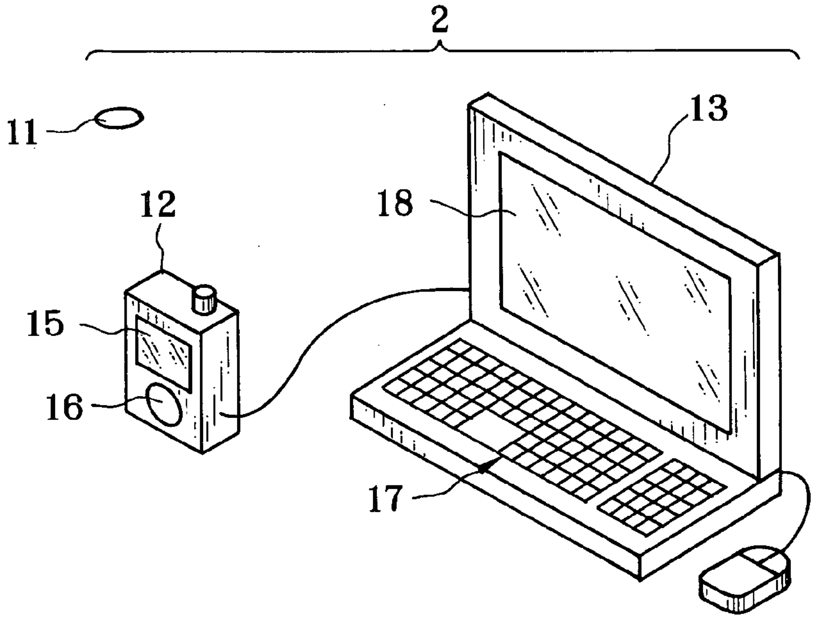

FIG. 2

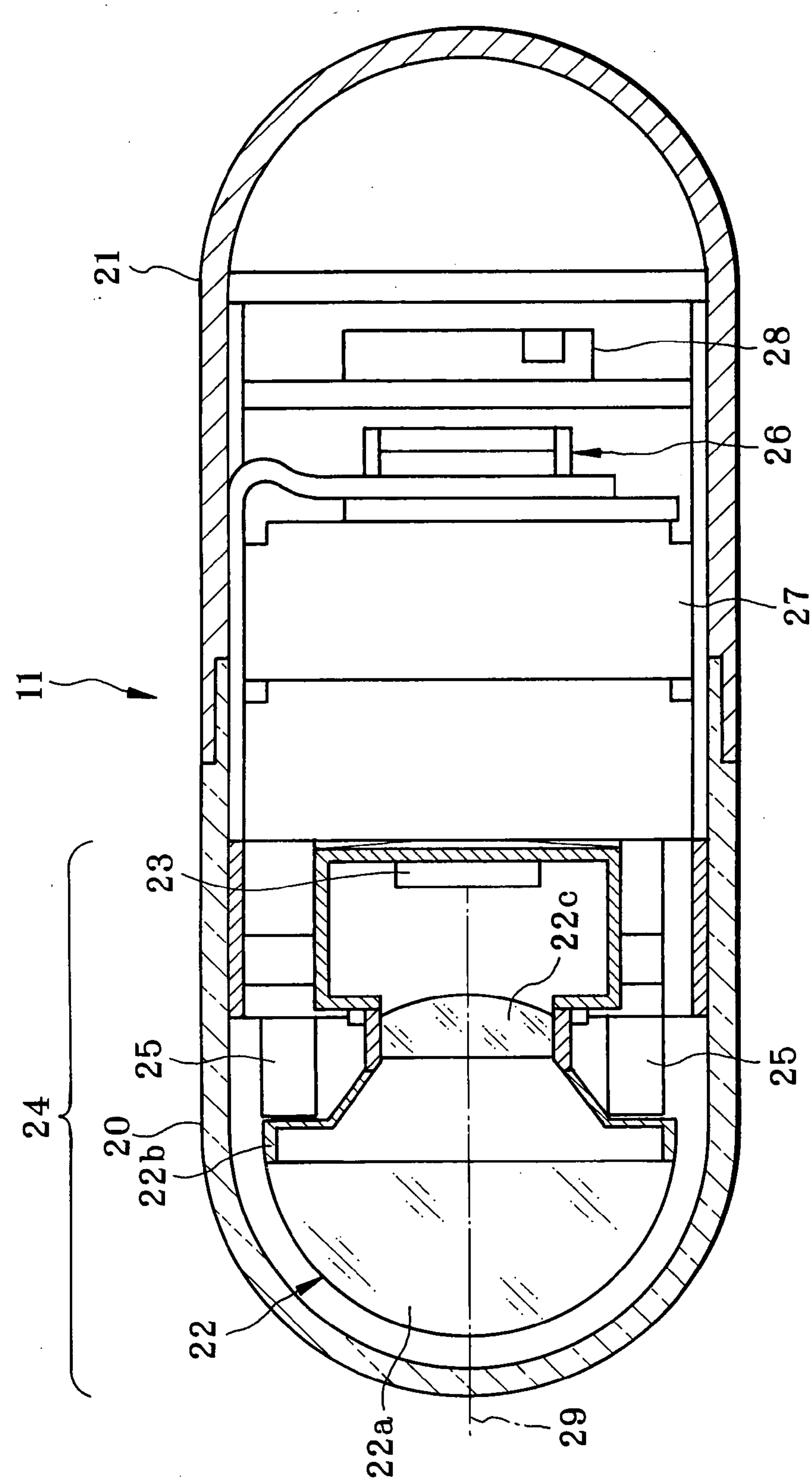

FIG. 3

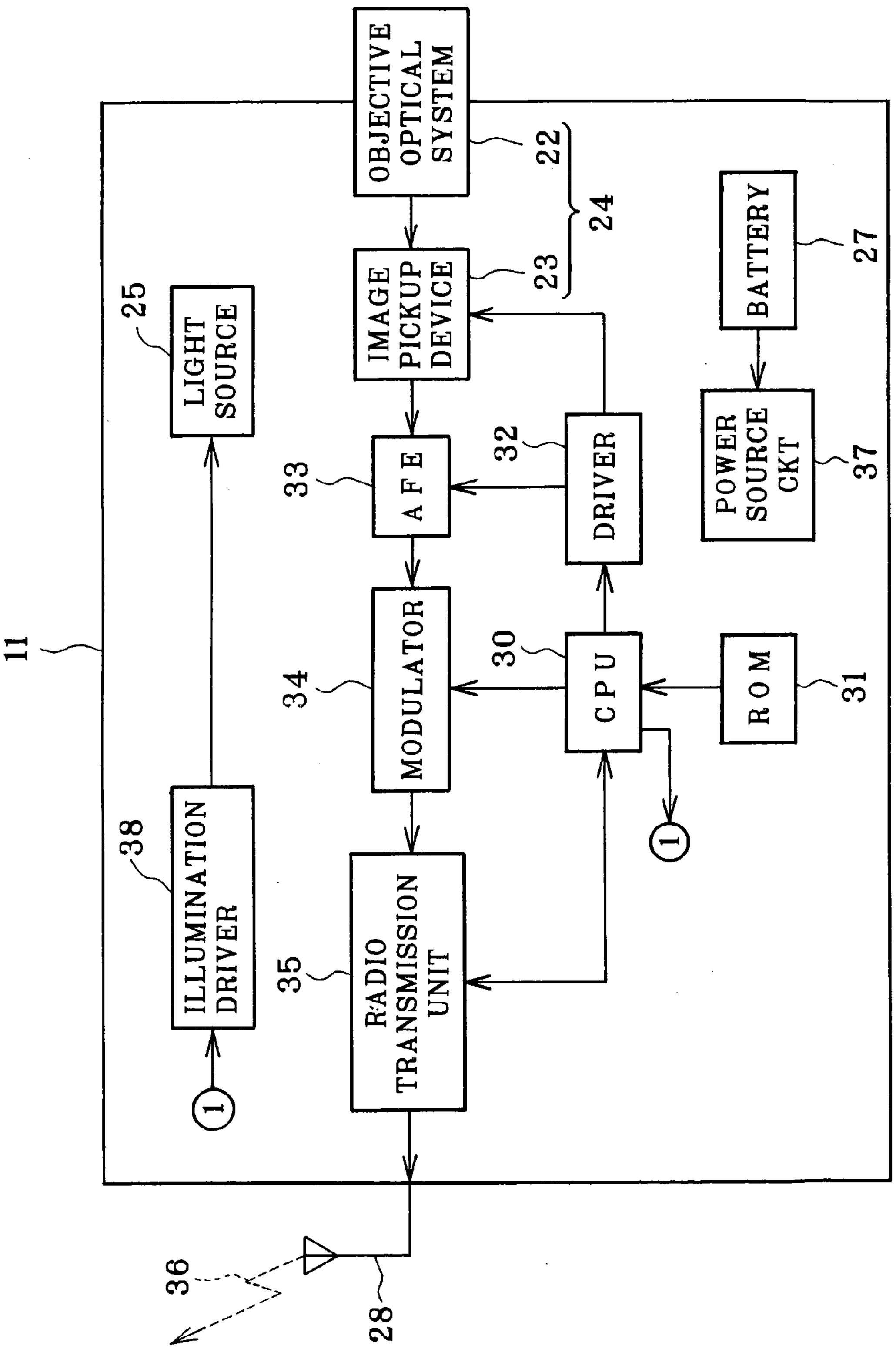
11
OBJECTIVE OPTICAL SYSTEM
22
24
23
IMAGE PICKUP DEVICE
LIGHT SOURCE
25
BATTERY
27
POWER SOURCE CKT
37
DRIVER
32
AFE
33
MODULATOR
34
CPU
30
ROM
31
1
ILLUMINATION DRIVER
38
RADIO TRANSMISSION UNIT
35
1
36
28

FIG. 4

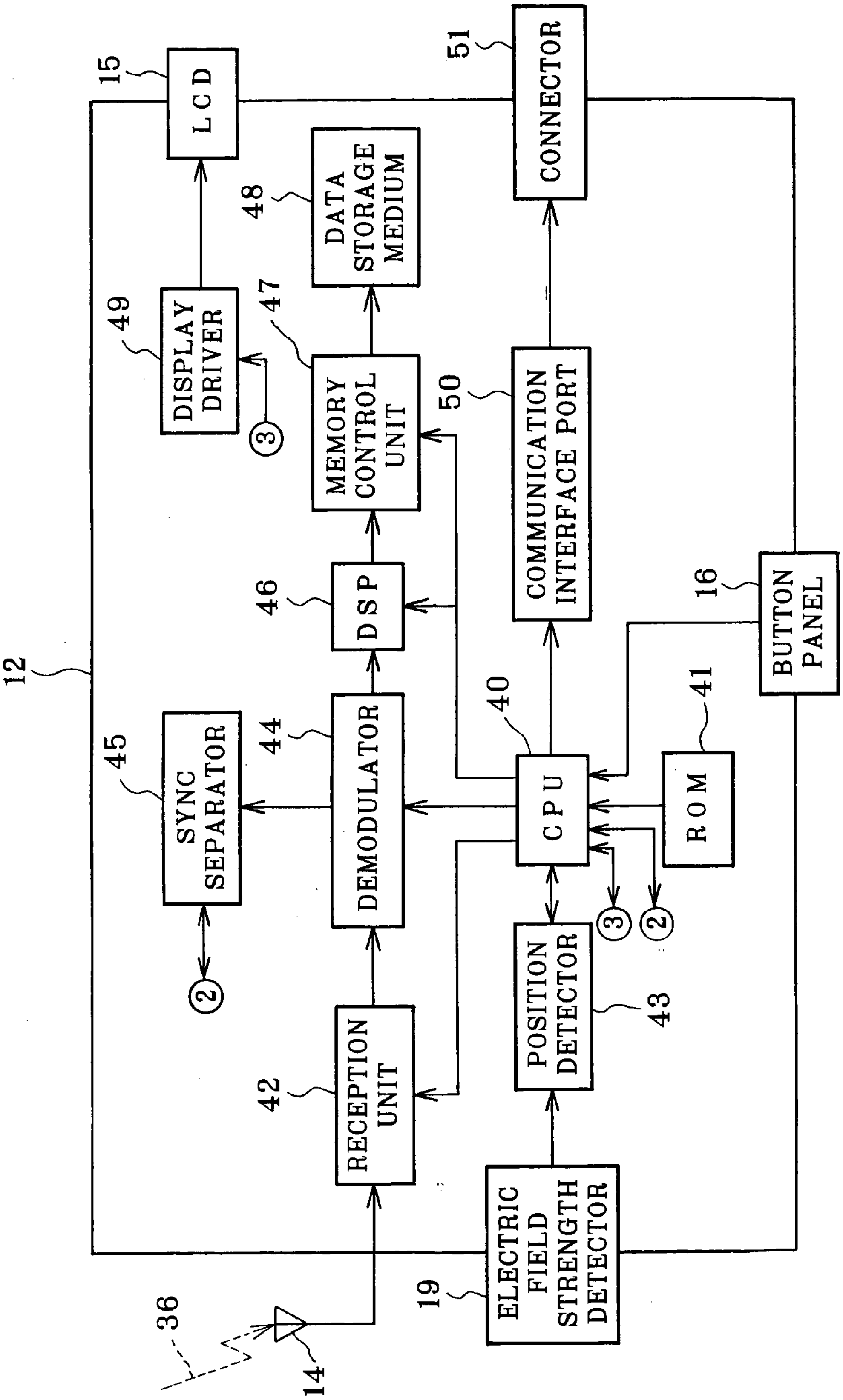
LCD
DATA STORAGE MEDIUM
CONNECTOR
DISPLAY DRIVER
MEMORY CONTROL UNIT
COMMUNICATION INTERFACE PORT
DSP
BUTTON PANEL
SYNC SEPARATOR
DEMODULATOR
CPU
ROM
POSITION DETECTOR
RECEPTION UNIT
ELECTRIC FIELD STRENGTH DETECTOR
12
14
15
16
19
36
40
41
42
43
44
45
46
47
48
49
50
51

FIG. 5

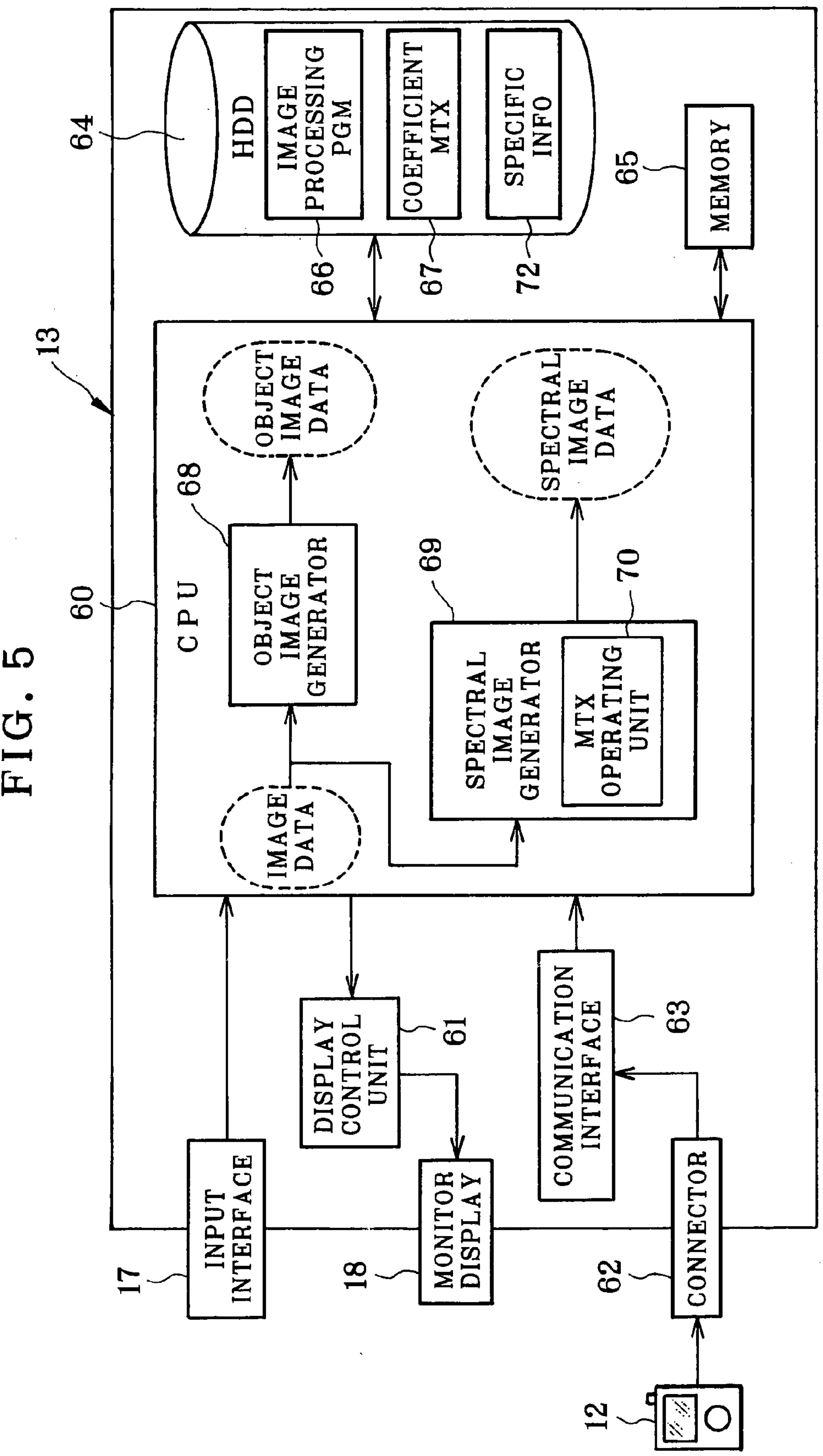

13
64
HDD
IMAGE PROCESSING PGM
COEFFICIENT MTX
SPECIFIC INFO
66
67
72
65
MEMORY
60
CPU
68
OBJECT IMAGE GENERATOR
OBJECT IMAGE DATA
69
SPECTRAL IMAGE GENERATOR
70
MTX OPERATING UNIT
SPECTRAL IMAGE DATA
IMAGE DATA
DISPLAY CONTROL UNIT
61
COMMUNICATION INTERFACE
63
17
INPUT INTERFACE
18
MONITOR DISPLAY
62
CONNECTOR
12

FIG. 6

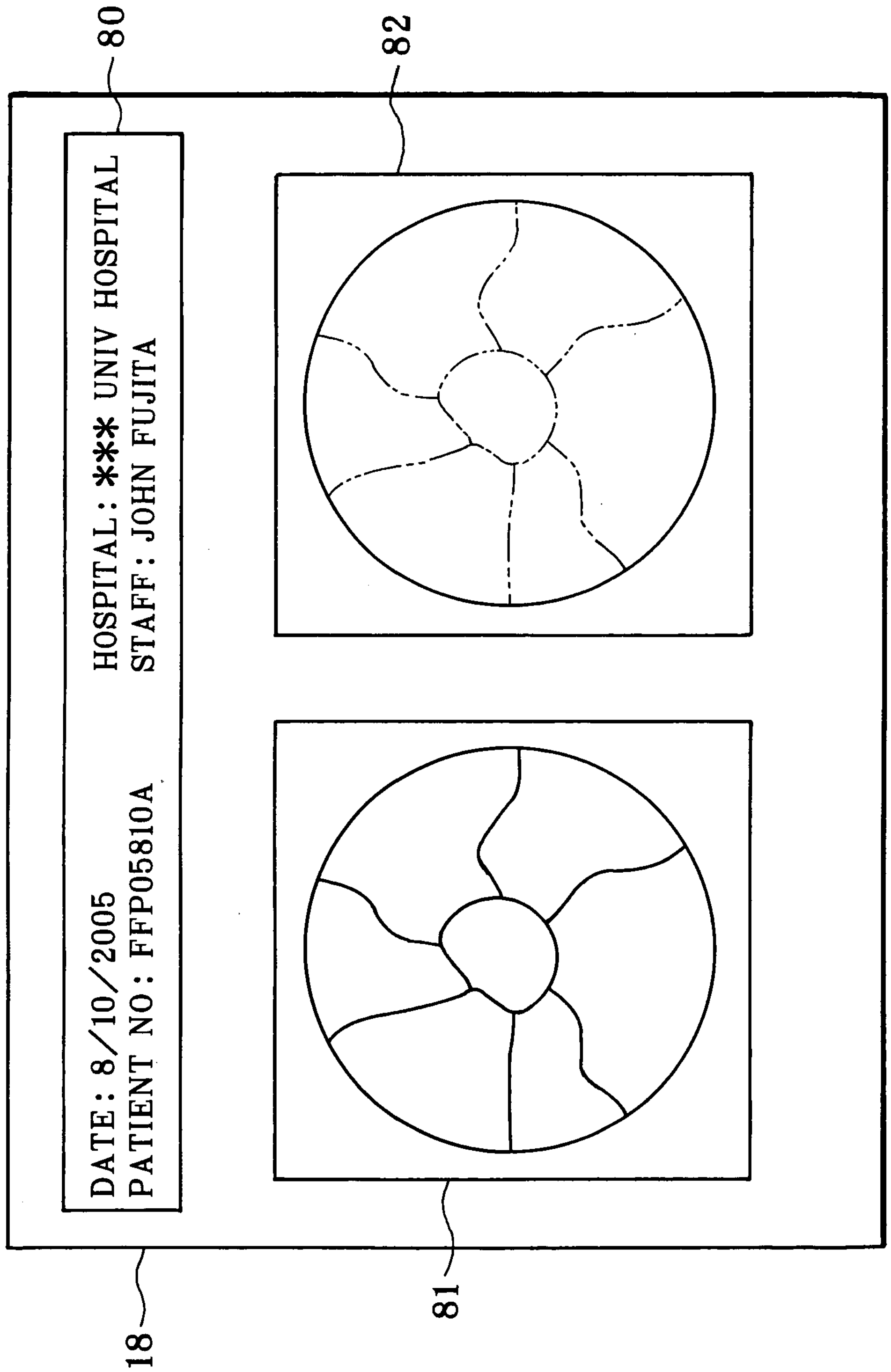
DATE: 8/10/2005
PATIENT NO: FFP05810A
HOSPITAL: *** UNIV HOSPITAL
STAFF: JOHN FUJITA
80
82
81
18

FIG. 7

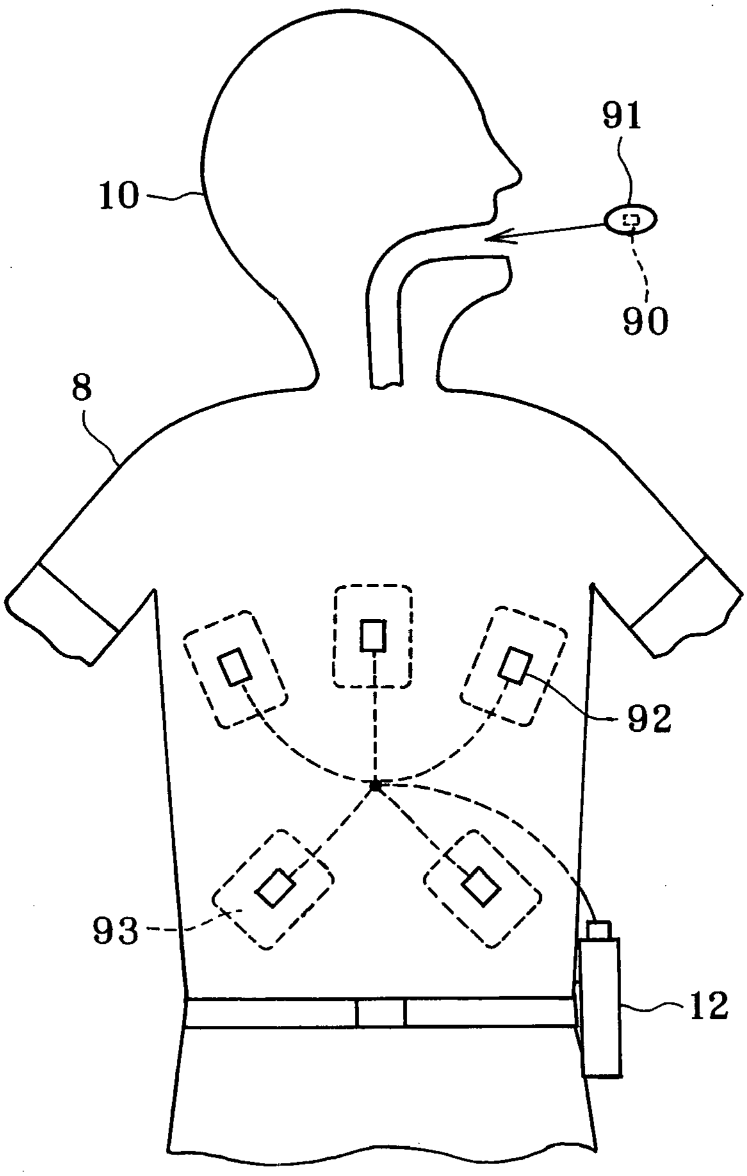

FIG. 8

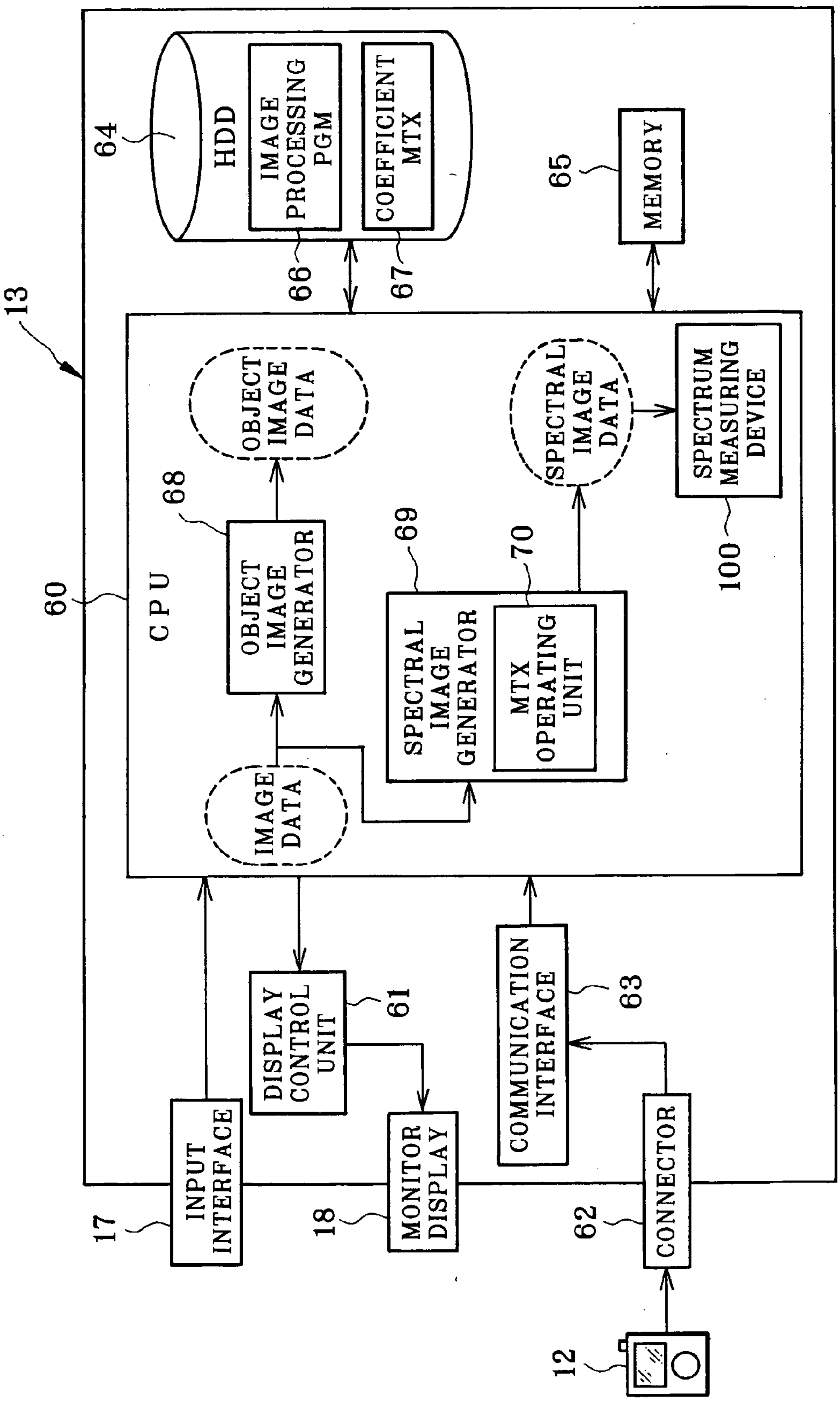
13
64
HDD
IMAGE PROCESSING PGM
COEFFICIENT MTX
66
67
65
MEMORY
60
CPU
OBJECT IMAGE DATA
68
OBJECT IMAGE GENERATOR
IMAGE DATA
69
SPECTRAL IMAGE GENERATOR
70
MTX OPERATING UNIT
SPECTRAL IMAGE DATA
SPECTRUM MEASURING DEVICE
100
DISPLAY CONTROL UNIT
61
COMMUNICATION INTERFACE
63
17
INPUT INTERFACE
18
MONITOR DISPLAY
62
CONNECTOR
12

FIG. 9

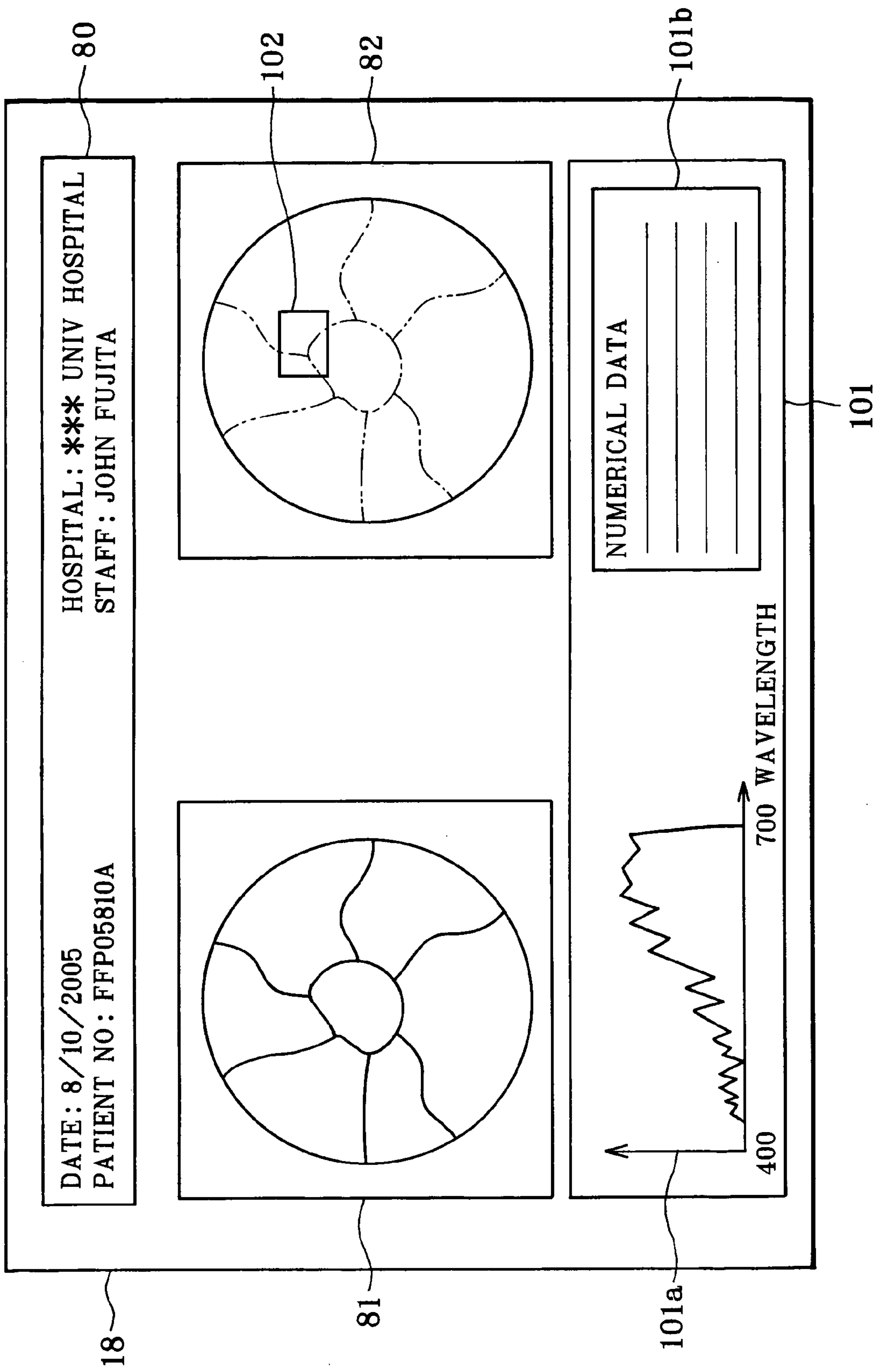
80
102
82
101b
HOSPITAL: *** UNIV HOSPITAL
STAFF: JOHN FUJITA
DATE: 8/10/2005
PATIENT NO: FFP05810A
NUMERICAL DATA
400
700
WAVELENGTH
101
18
81
101a

FIG. 10

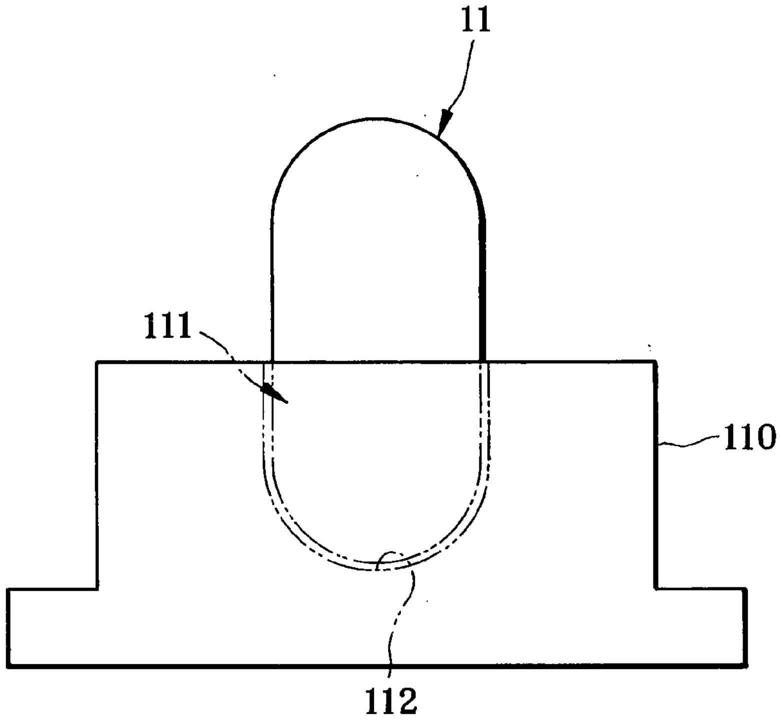


FIG. 11

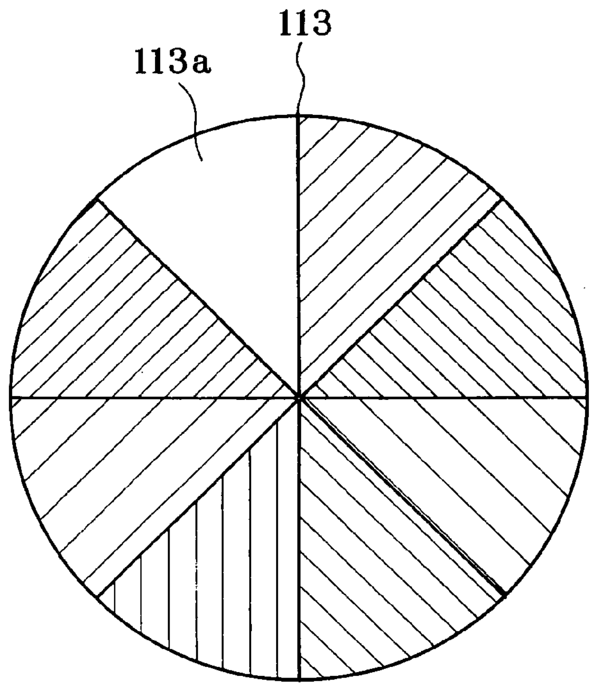

FIG. 12

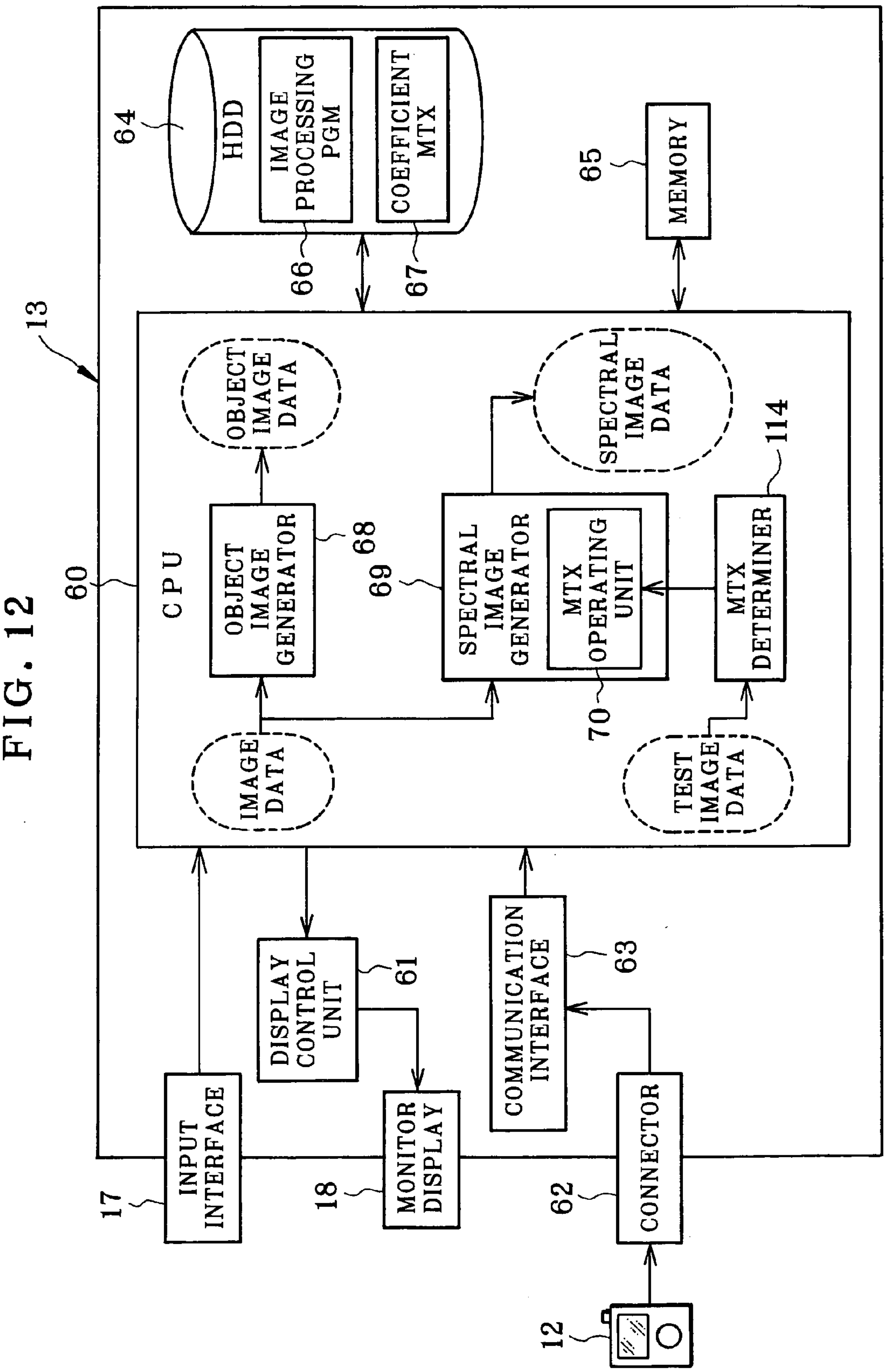
13
HDD
IMAGE PROCESSING PGM
COEFFICIENT MTX
64
66
67
MEMORY
65
CPU
60
OBJECT IMAGE DATA
OBJECT IMAGE GENERATOR
68
SPECTRAL IMAGE DATA
SPECTRAL IMAGE GENERATOR
69
MTX OPERATING UNIT
70
MTX DETERMINER
114
IMAGE DATA
TEST IMAGE DATA
INPUT INTERFACE
17
DISPLAY CONTROL UNIT
61
MONITOR DISPLAY
18
COMMUNICATION INTERFACE
63
CONNECTOR
62
12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7153259 B
- JP 2005074034 A